# EUROPÄISCHE PATENTSCHRIFT

(11) **EP 3 990 072 B1**
(45) Veröffentlichungstag und Bekanntmachung des Hinweises auf die Patenterteilung: **13.05.2026**
(21) Anmeldenummer: 20736919.0
(22) Anmeldetag: 15.06.2020
(51) Int. Cl.: A61M 16/00, A61J 15/00

(54) **SPERRELEMENT**
BLOCKING ELEMENT
ÉLÉMENT DE BLOCAGE

(30) Priorität: 17.06.2019 DE 102019116404
(43) Veröffentlichungstag der Anmeldung: 04.05.2022
(73) Patentinhaber: Fusch, Christoph, 90537 Feucht (DE)
(72) Erfinder: Fusch, Christoph, 90537 Feucht (DE)
(74) Vertreter: Tergau & Walkenhorst Intellectual Property GmbH
(86) Internationale Anmeldenummer: PCT/EP2020/066505
(87) Internationale Veröffentlichungsnummer: WO 2020/254254

(56) Entgegenhaltungen:
- WO-A1-2017/036846
- DE-A1- 102015 103 155
- US-A1- 2008 154 191

## Beschreibung

Die Erfindung betrifft ein Sperrelement zur Unterdrückung des Eindringens und Ansammelns von Atemluft im Magen bei der künstlichen, nicht invasiven Beatmung, insbesondere nasalen CPAP-Beatmung.

Der medizinische Hintergrund für die Erfindung ist die Notwendigkeit zur Behandlung des Atemnotsyndroms bei Neugeborenen. Dieses Atemnotsyndrom, auch Surfactant-Mangelsyndrom genannt, tritt vorwiegend bei Frühgeborenen auf, dies um so häufiger und ausgeprägter, je unreifer die Kinder sind. Für eine normale Lungenfunktion ist Surfactant notwendig. Surfactant ist eine oberflaechenaktive Substanz, die die Oberflächenspannung der Alveolen verringert und auch moduliert. Sie wird von Pneumozyten Typ II produziert und legt sich als Film über die Oberfläche der Alveolen. Surfactant ermöglicht die Luftfüllung der Lunge mit normalen Drucken, verhindert den endexpiratorischen Kollaps der Alveolen, so dass sein ausreichendes funktionelles Residualvolumen verbleibt. Wegen der Modulation der Oberflächenspannung verhindert Surfactant auch eine Verteilungsstörung in der Lunge, die sonst Bereiche von kollabierten und überblähten Abschnitten aufweisen würde. Surfactant wird in der Regel erst ab der 34. Schwangerschaftswoche ausreichend gebildet. Deswegen sind Frühgeborene vom Atemnotsyndrom durch Surfactantmangel häufiger betroffen. Es können aber auch reifere Kinder durch eine sekundäre Inaktivierung daran erkranken.

Die Behandlung des Atemnotsyndromes erfordert in der Regel eine Form von Beatmung bzw. Atemunterstützung. Man unterscheidet invasive und nicht-invasive Beatmungsformen. Bei den invasiven Prozeduren wird in der Regel ein Tubus durch Mund oder Nase endotracheal in die Luftröhre eingeführt. Der Gasaustausch erfolgt durch ein angeschlossenes Beatmungsgerät, das wie eine Art medizinische Luftpumpe per Überdruck ein Luft-Sauerstoffgemisch in die Lunge einbringt. Die Ausatmung erfolgt entweder passiv oder wie bei der Hochfrequenzbeatmung auch aktiv durch Unterdruckanwendung. Neben der Erzielung eines ausreichenden Gasaustausches ist es wichtig, dass ein kontinuierlicher distendierender Druck appliziert wird (positive end-expiratory pressure, PEEP). Dieser hält die Alveolen am Ende der Expiration offen und bewirkt das für das Überleben wichtige Funktionelle Residualvolumen. Dieses stellt sicher, dass bei auch unreifen Kindern der Austausch von Sauerstoff und Kohlendioxid während der relativ längeren Exspiration stattfindet. Seit einigen Jahren weiß man aber auch, dass eine invasive Beatmung nicht immer notwendig ist, sondern dass für viele Kinder eine nicht-invasive Atemunterstützung vollkommen ausreicht. Bei der nicht-invasiven Beatmung wird das Luft-Sauerstoff-Gemisch mit einem bestimmten Druck über eine nasale Kanüle oder Maske in den Nasen-Rachen-Raum appliziert. Bei erhaltener Eigenatmung setzen sich dieser Druck und Gasfluss intrapulmonal fort und erleichtern die Inspiration sowie die Aufrechterhaltung des Funktionellen Residualvolumens. Diese Beatmungsform wird CPAP ("continuous positive airway pressure") genannt und der wichtigste Parameter hierbei ist wiederum der bereits zuvor erwähnte PEEP. Viele Studien haben heute gezeigt, dass nicht-invasive CPAP-Beatmung der invasiven Beatmung ebenbürtig und in einigen Aspekten überlegen ist. In den Industrieländern werden mittlerweile mehr als 50% der sehr unreifen Frühgeborenen ausschließlich mit CPAP behandelt In der praktischen Durchführung kann es allerdings bei der CPAP-Anwendung zu Nebeneffekten kommen, die klinisch signifikant bzw. limitierend wirken können. Bei sehr unreifen Frühgeborenen stellt das Auftreten eines sogenannten "CPAP belly" ein Problem dar.

Die Atemluft gelangt sowohl durch die Nase als auch den Mund zunächst in den Rachenraum. Im Rachenraum beginnt nicht nur die Luftröhre, in welche die Atemluft gelangen soll, sondern neben dem Luftröhreneingang ist auch der Speiseröhreneingang angeordnet. Bei einer nicht invasiven CPAP-Beatmung mit Maske tritt das Problem auf, dass die mit Überdruck intubierte Luft nicht nur durch die Luftröhre in die Lunge sondern auch durch die Speiseröhre in den Magen gelangt. Der Magen wird so kontinuierlich aufgepumpt, was unerwünscht ist. Vor allem drückt ein aufgepumpter Magen von unten her gegen die Lunge und verringert die funktionelle Residualkapazität. Zusätzlich erschwert dieser das Einatmen in die Lunge und das Ausatmen aus der Lunge. Von Seiten der Magen-Darm-Funktion kann diese fehlerhaft zugeführte Luft ebenfalls zu Unverträglichkeiten der enteral zugeführten Nahrung führen. Dies kann in einer Reduktion der Nahrungszufuhr münden, was ihrerseits einen längerdauernden parenteralen Nahrungszugang erfordert und daher neben insuffizientem Wachstum auch zu einer höheren Sepsisrate führen kann. Insgesamt ist also der nicht beabsichtige Lufteintrag durch CPAP in den Magen-Darm-Trakt ein Problem von größerer klinischer Bedeutung.

Aus der WO2017/036846 ist eine Magensonde mit einer in der Speiseröhre angeordneten Manschette (Cuff) bekannt. Diese Magensonde weist einen im Rachenraum des Patienten platzierten Absaugabschnitt zur Absaugung von Speichel oder Sekret auf, um zu verhindern, dass Speichel oder Sekret in die Luftröhre eindringt. Der Speichel wird deshalb vor dem Erreichen der Luftröhre über den Absaugabschnitt abgeführt. Wird ein Patient jedoch durch den Rachenraum mit Überdruck beatmet, ist ein im Rachenraum angeordneter Absaugabschnitt völlig ungeeignet.

Ausgehend hiervon liegt der Erfindung die Aufgabe zugrunde, ein Sperrelement zu schaffen, welches ein Eindringen der mit Überdruck versehenen Atmungsluft aus dem Nasen-Rachenraum in den Magen wirksam verhindert, ohne eine Nahrungsaufnahme des Patienten zu behindern und zugleich zu verhindern, dass der Patient an in der Speiseröhre angesammeltem Speichel erstickt.

Das Sperrelement mit den Merkmalen des Anspruchs 1 löst diese Aufgabe in erfinderischer Weise. Die rückbezogenen Ansprüche lehren teilweise vorteilhafte und teilweise für sich selbst erfinderische Weiterbildungen dieser Erfindung.

Die Erfindung beruht auf der Grundüberlegung, die Speiseröhre zu verschließen, um ein Eindringen der Atemluft mit PEEP-Überdruck in den Magen von vorneherein auszuschließen. Um den Patienten gleichwohl zu ernähren, besteht das Sperrelement aus einer die Speiseröhre durchsetzenden, von einem dünnen Schlauch gebildeten, Magensonde. Diese Magensonde ist an ihrer Außenseite von einer aufblasbaren Manschette flankiert. Eine derartige Manschette wird auch als "Cuff" bezeichnet. Die aufgeblasene Manschette bildet in aufgeblasenem Zustand einen balonförmigen Pfropfen in der Speiseröhre. Der balonförmige Pfropfen verschließt im aufgeblasenen Zustand die Speiseröhre vollständig und liegt mit seiner Außenwand an den Innenwänden der Speiseröhre an. Infolge der Kompressibilität der Luft, kann sich die balonartige Manschette an die Innenwände der Speiseröhre gleichsam anschmiegen.

Zur Ernährung des Patienten ist - wie bereits ausgeführt - die schlauchförmige Magensonde durch die Manschette (Cuff) hindurch geleitet.

Bei dieser Anordnung besteht jedoch das Problem, dass die aufgeblasene Manschette nicht nur den Durchtritt der mit PEEP-Überdruck versehenen Atemluft verhindert sondern auch den üblichen Speichelabfluss vom Rachen durch die Speiseröhre hindurch in den Magen. Dieser durchbrochene Speichelabfluss in den Magen zieht dann einen Rückstau des Speichels oberhalb der aufgeblasenen Manschette nach sich. Im Extremfall sammelt eine so große Speichelmenge an, dass der Speichel von der aufgeblasenen Manschette her in den Rachenraum zurückfließt und im Extremfall in die Luftröhre eindringt.

Zur Verhinderung eines derartigen Speichelrückstaus weist das erfindungsmäßige Sperrelement eine Vorrichtung zum Abtransport des in die Speiseröhre eindringenden Speichels auf. Mit einer derartigen Vorrichtung kann der sich in der Speiseröhre ansammelnde Speichel entweder kontinuierlich oder in gewissen Zyklen abtransportiert werden.

Gemäß der Erfindung weist das erfindungsmäßige Sperrelement eine weitere Absaugsonde als Vorrichtung zum Abtransport des Speichels auf. Diese Absaugsonde ist - ebenso wie die Magensonde - schlauchförmig ausgebildet. Mit ihrem freien Ende endet die Absaugsonde oberhalb der Manschette. Im Bereich ihres oberhalb der Manschette angeordneten Freiendes weist die Absaugsonde in ihrem Schlauchmantel eine Vielzahl von Absaugöffnungen auf, um den von der Manschette zurückgestauten Speichel aufnehmen und abtransportieren zu können. Die Vielzahl von Absaugöffnungen verhindert auch ein Anliegen oder Festsaugen der Außenwand der Absaugsonde an der Innenwand der Speiseröhre.

Im Bereich dieser Absaugöffnungen kann ein zusätzlicher Abstandhalter oder Käfig angeordnet sein, der an der Innenwand der Speiseröhre mit seiner Außenwand anliegt. Dieser Abstandhalter oder Käfig hält die Speiseröhreninnenwand und die Ansaugöffnungen auf Distanz. Die Ansaugöffnungen bleiben so frei und funktionstüchtig.

In einer Ausführungsform dieser Vorrichtung ist hierfür die in der Speiseröhre angeordnete aufblasbare und die Speiseröhre im aufgeblasenen Zustand verschließende Manschette so ausgestaltet, dass sie sich intermittierend mit Luft füllt und in gewissen Zeitabständen die Luft wieder ausbläst. Die balonartige Manschette wird also in vordefinierten Intervallen mit Luft gefüllt und anschließend wieder entleert. Die Entleerungsintervalle sind dabei im Vergleich zu den gefüllten Intervallen recht kurz. Sie reichen aber aus, um die gesammelte Speichelmenge an den Außenwänden der Manschette bzw. des Cuffs vorbeifließen zu lassen und direkt in den Magen einzuleiten. Bei entleerter Manschette drückt der PEEP-Überdruck den Speichel gleichsam an den Außenwänden der Manschette bzw. des Cuffs vorbei in den Magen.

In einer anderen Ausführungsform der Erfindung sind die beiden vorgenannten Vorrichtungen zum Abtransport des Speichels miteinander kombiniert. Zusätzlich zur intermittierend mit Luft befüllten und wieder entleerten Manschette ist eine Absaugsonde bei dieser Ausführungsform der Erfindung vorgesehen. Die nachfolgende Beschreibung bezieht sich sowohl auf die jeweilige Ausführungsform in Alleinstellung als auch auf die kombinierte Vorrichtung.

In vorteilhafter Ausgestaltung der Erfindung ist die Manschette, also der Cuff, im Bereich der Cardia am Ende der Speiseröhre angeordnet. Sowohl die Magensonde als auch die Absaugsonde durchsetzen bei dieser Ausführungsform die Speiseröhre vollständig. Die balonartige Manschette verschließt somit den Magen im Bereich seines Mageneingangs. Diese Anordnung hat den Vorteil, dass ein eventuell verbleibender Reststau von Speichel im Bereich der Manschette rachenfern auftritt, so dass ein unerwünschtes Eindringen des Speichels in die Luftröhre faktisch ausgeschlossen ist.

In einer weiteren alternativen Ausführungsform ist die Manschette im oberen Mediastinum der Speisröhre angeordnet. Auf diese Weise ist sichergestellt, dass die Absaugsonde keinen Speichel aus dem Rachenraum und aus dem oberhalb des Mediastinums angeordneten Halsteil der Speisröhre (pars cervicalis) absaugt. Der Rachenraum und der Halsteil der Speiseröhre sollen nämlich durchaus mit Speichel versorgt sein, um ein Austrocknen des Rachenraums zu verhindern. Ein Austrocknen des Rachenraums könnte ungewollte Hustenanfälle oder ähnliche andere ungewünschte Reaktionen des Patienten, insbesondere eines Neugeborenen, nach sich ziehen.

Die bevorzugte Anordnung der Manschette im oberen Mediastinum hat ferner den Vorteil, dass der Absaugweg durch die Absaugsonde vergleichsweise kurz ist.

Alternativ dazu kann die Manschette auch im hinteren Mediastinum unterhalb des oberen Mediastinums angeordnet sein. Diese Anordnung vermeidet einerseits zu lange Absaugwege der Absaugsonde und gewährleistet andererseits eine ausreichende Distanz der Manschette zum Rachenraum.

An dem dem freien Ende der Absaugsonde abgewandten Ende ist ein Sammelgefäß vorgesehen. In diesem Sammelgefäß wird der abgesaugte Speichel zwischengespeichert und über einen mit der Magensonde leitungsmäßig verbundenen Mehrwegehahn in die Magensonde schlussendlich eingebracht, um durch die Magensonde in den Magen eingeleitet zu werden. Diese Einleitung des angesammelten Speichels hat den Vorteil, dass nahezu die gesamte vom Körper produzierte Speichelmenge für den Verdauungsvorgang im Magen zur Verfügung steht.

Anhand des in der Zeichnungsfigur dargestellten Ausführungsbeispiels ist die Erfindung näher erläutert:

Die Zeichnungsfigur zeigt schematisch einen Abschnitt einer Speiseröhre 1. In der Speiseröhre 1 liegt die aufgeblasene Manschette 2 (= Cuff) ein. Die balonartige Manschette 2 liegt mit ihren Außenwänden an den Innenwänden der Speiseröhre 1 an und verschließt die Speiseröhre 1 so. Durch die Manschette 2 ist die Magensonde 3 hindurch geleitet. Die Magensonde 3 endet im schematisch angedeuteten Magen 4. In der Zeichnungsfigur angedeutet ist die Cardia 5. Die Zeichnungsfigur zeigt also eine im hinteren Mediastinum angeordnete Manschette 2.

Oberhalb der Manschette 2 endet das Freiende der Absaugsonde 6. Im Bereich des Freiendes der Absaugsonde 6 sind in den Schlauchmantel der Absaugsonde 6 Absaugöffnungen 7 eingebracht. Über die Absaugöffnungen 7 und eine weitere Öffnung im freien Ende der Absaugsonde 6 kann der an der Manschette 2 zurückgestaute Speichel abgesaugt werden.

Beim Ausführungsbeispiel wird der abgesaugte Speichel durch die Absaugsonde 6 in das Sammelgefäß 8 eingeleitet. Aus dem Sammelgefäß 8 kann der im Sammelgefäß 8 gesammelte Speichel über einen Mehrweghahn 9 in die Magensonde 3 eingeleitet werden. Durch die Magensonde 3 kann der Speichel durch die Manschette 2 hindurch in den Magen 4 eingeleitet werden.

In der Zeichnungsfigur ist schließlich eine kombinierte Zu- und Abluftleitung 10 erkennbar. Die Zu- und Abluftleitung 10 ist an ihrem Ende mit einem Kompressor 11 verbunden. Mit Hilfe des Kompressors 11 wird die Luft zum Aufblasen der Manschette 2 bzw. des Cuffs eingeleitet. Über ein in der Zu- und Abluftleitung so weiterhin angeordnetes Ventil 12 kann die in der Manschette 2 bzw. im Cuff gesammelte Luft wieder aus dem System ausgeleitet werden. Auf diese Weise kann durch abwechselndes Betätigen des Kompressors 11 und des Ventils 12 ein intermittierender Betrieb der Manschette 2 bzw. des Cuffs realisiert werden. Die Manschette 2 wird aufgeblasen, um die Speiseröhre 1 zu verschließen. Der Speichelfluss durch die Speiseröhre 1 in den Magen 4 ist dann gesperrt bzw. gehemmt. Wird die Luft durch das Ventil 12 wieder aus der Manschette 2 ausgelassen, kann Speichel an den Außenwänden der Manschette 2 vorbei durch die Speiseröhre 1 fließen und in den Magen 4 gelangen.

Auf diese Weise ist es wirksam verhindert, dass Speichel in die neben der Speiseröhre 1 verlaufende, in den Zeichnungsfiguren nicht dargestellte, Luftröhre eindringt.

### Bezugszeichenliste

- 1: Speiseröhre
- 2: Manschette
- 3: Magensonde
- 4: Magen
- 5: Cardia
- 6: Absaugsonde
- 7: Absaugöffnung
- 8: Sammelgefäß
- 9: Mehrweghahn
- 10: Zug- und Abluftleitung
- 11: Kompressor
- 12: Ventil Zusammenfassung

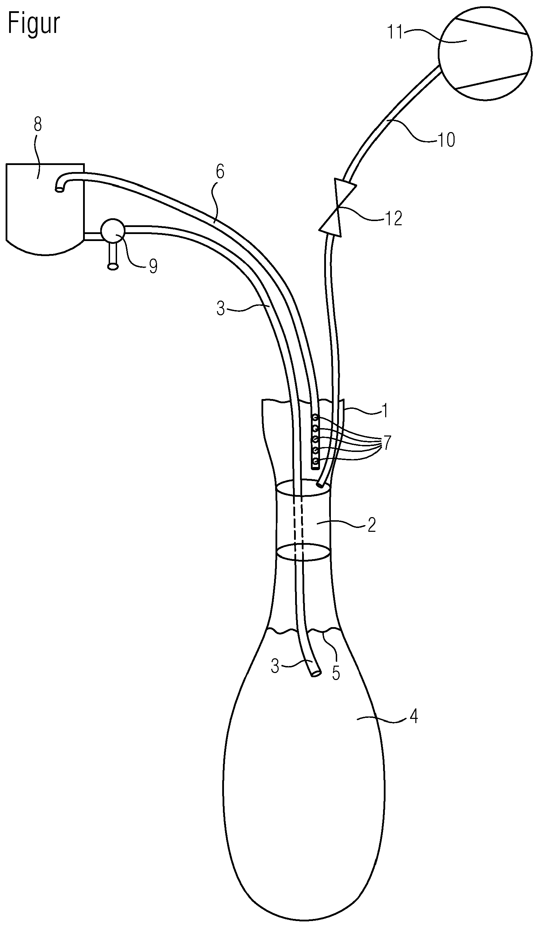

## Patentansprüche

1. Sperrelement zur Unterdrückung des Eindringens und Ansammelns von Atemluft im Magen (4)
mit einer die Speiseröhre (1) durchsetzenden, von einem Schlauch gebildeten Magensonde (3),
mit einer, mit Luft aufblasbaren und die Speiseröhre (1) im aufgeblasenen Zustand verschließenden Manschette (2) und
mit einer Vorrichtung zum Abtransport von in die Speiseröhre (1) eindringendem Speichel,
**dadurch gekennzeichnet, dass** die Vorrichtung eine in die Speiseröhre (1) hineinragende und mit ihrem freien Ende an der Manschette (2) endende, als Absaugschlauch ausgebildete Absaugsonde (6) für in die Speiseröhre (1) eindringenden Speichel mit Absaugöffnungen (7) in den in der Speiseröhre (1) einliegenden Bereichen des Schlauchmantels umfasst
und **gekennzeichnet durch** ein Sammelgefäß (8) für den mit der Absaugsonde (6) abgesaugten Speichel an dem dem freien Ende der Absaugsonde (6) abgewandten Sondenausgang und durch einen mit der Magensonde (3) leitend verbundenen Mehrweghahn (9) zur Einleitung des im Sammelgefäß (8) gesammelten Speichels durch die Magensonde (3) in den Magen (4).

2. Sperrelement nach Anspruch 1,
**dadurch gekennzeichnet,**
**dass** die Manschette (2) dazu ausgebildet ist intermittierend entleert und anschließend wieder gefüllt zu werden, um als kombinierte Vorrichtung zum Abtransport von in die Speiseröhre eindringendem Speichel zu dienen.

3. System nach Anspruch 1 oder 2
**dadurch gekennzeichnet,**
**dass** die Manschette (2) im Bereich der Cardia (5) am Ende der Speiseröhre (1) angeordnet ist.

4. System nach Anspruch 1, 2 oder 3
**dadurch gekennzeichnet,**
**dass** die Manschette (2) im oberen Mediastinum angeordnet ist.

## Claims

1. Blocking element for suppressing the ingress and accumulation of respiratory air in the stomach (4),
with a gastric probe (3) formed by a tube which extends through the oesophagus (1),
with a cuff (2) which can be inflated with air and which closes the oesophagus (1) in the inflated state, and
with a device for removing saliva penetrating into the oesophagus (1),
**characterized in that** the device
comprises a suction probe (6) for saliva penetrating into the oesophagus (1) and constructed as a suction tube which protrudes into the oesophagus (1) and with its free end terminating at the cuff (2), with suction openings (7) in the regions of the tube sheath located in the oesophagus (1),
and **characterized by** a collecting vessel (8) for the saliva aspirated with the suction probe (6) at the probe outlet facing away from the free end of the suction probe (6) and by a selector valve (9) which is conductively connected to the gastric probe (3) for introducing the saliva collected in the collecting vessel (8) into the stomach (4) through the gastric probe (3).

2. Blocking element as claimed in claim 1,
**characterized in that**
the cuff (2) is constructed to be intermittently emptied and subsequently refilled in order to serve as a combined device for removing saliva penetrating into the oesophagus.

3. System as claimed in claim 1 or claim 2,
**characterized in that**
the cuff (2) is disposed in the region of the cardia (5) at the end of the oesophagus (1).

4. System as claimed in claim 1, claim 2 or claim 3,
**characterized in that**
the cuff (2) is disposed in the superior mediastinum.

## Revendications

1. Élément de blocage pour supprimer la pénétration et l'accumulation d'air respirable dans l'estomac (4)
avec une sonde gastrique (1), formée par un tube traversant l'œsophage (3),
avec un brassard gonflable à l'air et fermant l'œsophage (1) à l'état gonflé (2) et
avec un dispositif permettant d'éliminer la salive pénétrant dans l'œsophage (1),
**caractérisé en ce que** le dispositif
comprend une sonde d'aspiration (6) qui pénètre dans l'œsophage (1) et se termine par son extrémité libre au niveau du brassard (2), réalisée sous forme d'un tuyau d'aspiration pour la salive pénétrant dans l'œsophage (1), et comportant des orifices d'aspiration (7) dans les zones situées dans l'œsophage (1)
et **caractérisé par** un récipient de collecte (8) pour la salive aspirée par la sonde d'aspiration (6) à l'extrémité libre de la sonde d'aspiration (6), et par un robinet à distribution multiple (9) relié conductivement à la sonde gastrique (3) pour introduire la salive recueillie dans le récipient de collecte (8) dans l'estomac (4).

2. Élément de blocage selon la revendication 1,
**caractérisé en ce que**
le brassard (2) est conçu pour être vidé par intermittence puis rempli à nouveau pour servir de dispositif combiné pour l'évacuation de la salive pénétrant dans l'œsophage.

3. Système selon la revendication 1 ou 2, **caractérisé en ce que**
le brassard (2) est placé dans la zone de la cardia (5) à l'extrémité de l'œsophage (1).

4. Système selon la revendication 1, 2 ou 3,
**caractérisé en ce que**
le brassard (2) est placé dans le médiastin supérieur.
